# EUROPÄISCHE PATENTSCHRIFT

(11) **EP 0 374 684 B1**
(45) Veröffentlichungstag und Bekanntmachung des Hinweises auf die Patenterteilung: **30.06.1993**
(21) Anmeldenummer: 89122878.5
(22) Anmeldetag: 12.12.1989
(51) Int. Cl.: G01N 33/543, G01N 33/558, G01N 33/538

(54) **Testträger zur analytischen Untersuchung einer Probenflüssigkeit mit Hilfe einer spezifischen Bindungsreaktion zweier bioaffiner Bindungspartner und entsprechendes Testverfahren**
Test strip for analysing a fluid-sample using a specific binding reaction between two bioaffinity binding partners, and a test method therefor
Bande d'essai pour l'analyse d'un échantillon fluide utilisant une réaction de liaison spécifique entre deux membres d'une réaction de liaison bioaffinés et méthode d'essai correspondante

(30) Priorität: 19.12.1988 DE 3842702
(43) Veröffentlichungstag der Anmeldung: 27.06.1990
(60) Teilanmeldung: 92117102.1
(73) Patentinhaber: BOEHRINGER MANNHEIM GMBH, 68298 Mannheim (DE)
(72) Erfinder: Schlipfenbacher, Reiner, Dr., D-6840 Lampertheim (DE); Mangold, Dieter, Dr., D-6701 Maxdorf (DE); Lerch, Rolf, Dipl.-Ing., D-6804 Ilvesheim (DE); Steinbiss, Joachim, Dr., D-6143 Lorsch (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 201 339
- EP-A- 0 225 054
- EP-A- 0 271 204
- EP-A- 0 271 854
- EP-A- 0 303 110
- WO-A-86/04683
- US-A- 4 189 304

## Beschreibung

Die Erfindung betrifft einen Testträger zur analytischen Untersuchung einer Probenflüssigkeit mit Hilfe einer spezifischen Bindungsreaktion zweier bioaffiner Bindungspartner, von denen einer in der Probe und einer in dem Reagenzsystem des Testträgers enthalten ist. Der Testträger hat mehrere auf einer Testschicht im wesentlichen nebeneinander angeordnete kapillaraktive Testzonen, die in Fluidkontakt zueinander stehen, so daß sie eine Flüssigkeitstransportstrecke bilden, entlang der eine Flüssigkeit durch Kapillarkräfte getrieben von einer Startzone zu einer Zielzone strömt. Dabei läuft zwischen dem ersten Bindungspartner und dem den zweiten Bindungspartner enthaltenden Reagenzsystem eine Nachweisreaktion ab, die zu einer für die gewünschte Analyse spezifischen markierten Spezies führt. Die markierte spezifische Spezies wird in einer Nachweiszone nachgewiesen.

Für qualitative und quantitative analytische Bestimmungen im Rahmen der Diagnose von Krankheiten werden in jüngerer Zeit zunehmend sogenannte trägergebundene Tests verwendet. Bei diesen Tests sind Reagenzien in entsprechende Schichten eines festen Testträgers eingebettet, der mit der Probe in Kontakt gebracht wird. Die Probe ist meist eine Körperflüssigkeit wie Blut oder Urin. Es kann sich aber auch um eine durch vorausgehende Testschritte gewonnene Flüssigkeit handeln.

Testträger sind in vielen verschiedenen Gestaltungen bekannt. Die Erfindung bezieht sich auf solche Testträger, bei denen kapillaraktive Testzonen, welche üblicherweise aus saugfähigen Schichtmaterialien wie Papieren, Vliesen oder porösen Kunststoffschichten bestehen, nebeneinander auf einer Basisschicht derart angeordnet sind, daß die Flüssigkeit parallel zu der Basisschicht die Flüssigkeitsstrecke entlangströmt. Man kann diese Testträger deshalb auch als "Testträger mit Längstransport" bezeichnen.

Derartige Testträgerkonstruktionen sind insbesondere für Analyseverfahren vorteilhaft, die auf einer spezifischen Bindungsreaktion zweier bioaffiner Bindungspartner beruhen. Beispiele sind in der deutschen Patentschrift 34 45 816 und in der US-Patentschrift 4 361 537 beschrieben. Spezifische Bindungsreaktionen in diesem Sinne sind insbesondere immunologische Interaktionen, also Wechselwirkungen zwischen Antigenen bzw. Haptenen einerseits und Antikörpern andererseits. Es können jedoch auch andere spezifische bioaffine Wechselwirkungen verwendet werden, wie Lectin-Zucker oder eine Wirkstoff-Rezeptor-Wechselwirkung. Im folgenden wird ohne Beschränkung der Allgemeinheit beispielhaft auf immunologische Wechselwirkungen Bezug genommen.

Es können sehr verschiedene Testprinzipien verwendet werden, die in der Literatur, beispielsweise in den beiden zitierten Patentschriften, beschrieben sind.

Eine erste Gruppe immunologischer Testführungen zeichnet sich dadurch aus, daß eine der vorderen Zonen der Flüssigkeitstransportstrecke den ersten Bindungspartner (Analyt) in löslicher markierter Form enthält. Eine im weiteren Verlauf der Flüssigkeitstransportstrecke angeordnete Testzone enthält trägerfixiert den zweiten Bindungspartner. Der Analyt aus der Probe und der markierte Analyt aus dem Test konkurrieren um die Bindungsstellen an dem zweiten Bindungspartner. Derartige Tests werden deshalb als kompetitive Tests bezeichnet.

Bei einer zweiten Gruppe bekannter immunologischer Testführungen enthält eine der vorderen Zonen der Flüssigkeitstransportstrecke den zweiten Bindungspartner in löslicher und markierter Form. Durch die spezifische Bindungsreaktion mit dem ersten Bindungspartner bilden sich bewegliche und markierte Komplexe.

Im weiteren Testverlauf kann ein weiterer Bindungspartner in trägerfixierter Form vorhanden sein, der mit einer durch die Komplexbildung nicht abgesättigten Bindungsstelle des ersten oder zweiten Bindungspartners spezifisch bindungsfähig ist. Dadurch entsteht ein Sandwich von mindestens drei Bindungspartnern. Man spricht deshalb auch von Sandwichtests.

Bei dem sogenannten immunenzymometrischen (IEMA) Prinzip enthält der weitere Testverlauf den Analyten in trägerfixierter Form. Dadurch wird der nicht komplexierte Anteil des zweiten Bindungspartners fixiert. Nur die Komplexe bleiben beweglich und können nachgewiesen werden.

Allen immunologischen Bestimmungen gemeinsam ist, daß die Nachweisreaktion, die unter anderem eine spezifische Bindungsreaktion zwischen den beiden Bindungspartnern umfaßt, zu einer für die gewünschte Analyse spezifischen markierten Spezies führt. Im Fall des kompetitiven Tests ist dies der gebundene oder der freigebliebene Analyt. Im Falle des Sandwichtests sind es die gebundenen Sandwichkomplexe. Im Falle des IEMA-Tests ist es der frei beweglich gebliebene Komplexe

Nähere Erläuterungen der verschiedenen bekannten immunologischen Testprinzipien sind nicht erforderlich, weil sie der einschlägigen Literatur entnommen werden können. Die Erfindung ist unabhängig vom speziellen Testverlauf vorteilhaft anwendbar, richtet sich jedoch insbesondere auf Testträger, die nach dem IEMA-Prinzip arbeiten.

Verschiedene immunologische Bestimmungsverfahren werden auch bezüglich der verwendeten Markierung unterschieden. Die Erfindung richtet sich insbesondere auf Enzymimmuntests, bei denen enzymatische Markierung verwendet wird. Das Markierungsenzym wird üblicherweise durch die farbbildende Reaktion eines Substrats des Markierungsenzyms nachgewiesen. Das Substrat kann - je nach Testführung - in der Nachweiszone bereits vorhanden sein oder dieser zugeführt werden. Die Erfindung ist grundsätzlich auch für nichtenzymatische Verfahren, bei denen beispielsweise ein Farbstoff oder ein radioaktives Element zur Markierung verwendet wird, anwendbar.

Um einen leicht handhabbaren und kostengünstig herstellbaren Testträger zur Verfügung zu stellen, der sich insbesondere für die Auswertung verhältnismäßig hoch konzentrierter Analyten eignet, für die immunologische Testträger bisher nicht zur Verfügung standen, schlägt die Erfindung vor, einen als Abfangreagenz wirkenden für den ersten Bindungspartner spezifischen dritten Bindungspartner vorzusehen, der in einer Zone der Flüssigkeitstransportstrecke so angeordnet und bezüglich der eingesetzten Menge so bemessen ist, daß er einen Teil des ersten Bindungspartners bindet und dadurch der zu der markierten Spezies führenden Nachweisreaktion entzieht. Dabei liegt die Zone mit dem Abfangreagenz zwischen der Startzone und der Nachweiszone.

Immunologische Bestimmungen zeichnen sich durch eine extrem hohe Empfindlichkeit aus. Dies ist für niedrig konzentrierte Analyten ein Vorteil, jedoch bestehen bereits seit längerem Bestrebungen, die Vorteile immunologischer Bestimmungen auch für höher konzentrierte Analyten einzusetzen. Dabei erweist sich die große Empfindlichkeit als schwieriges Problem. Selbstverständlich ist es möglich, die Probe manuell entsprechend zu verdünnen. Dies erfordert jedoch einen Handhabungsschritt, der nur von geschultem Laborpersonal ausgeführt werden kann und zeitaufwendig ist. Deswegen wurden Versuche gemacht, durch aufwendige Eingriffe in das Testverfahren dessen Empfindlichkeit zu reduzieren. Beispielsweise wurden im Test Bindungspartner verwendet, die eine reduzierte Affinität gegenüber dem Analyt aufweisen, oder es wurde die Stöchiometrie zwischen dem markierten Bindungspartner und dem Markierungsenzym manipuliert.

Die vorliegende Erfindung schlägt nun einen im Vergleich zu diesen bekannten aufwendigen Verfahren überraschend einfachen Weg vor, indem sie ein für den Analyten spezifisches Abfangreagenz auf dem Testträger vorsieht. Dadurch wird gewissermaßen eine Verdünnung der Analytenkonzentration auf dem Testträger erreicht, ohne daß ein separater vorgeschalteter Verdünnungsschritt notwendig ist. Der erfindungsgemäße Testträger ist für Konzentrationen über 10⁻⁸ mol/l, insbesondere über 10⁻⁷ mol/l zweckmäßig.

Ein Testträger mit Längstransport, bei dem am Anfang einer Flüssigkeitstransportstrecke ein für den Analytbindungspartner spezifischer dritter Bindungspartner zusätzlich zu einem enzymatisch markierten zweiten Bindungspartner vorgesehen ist, ist aus der EP-A-0 303 110 bekannt, welche einen Stand der Technik im Sinne von Artikel 54 (3) bildet. Der dritte Bindungspartner ist dort jedoch nicht in einer dem analytischen Nachweis dienenden Flüssigkeitstransportstrecke, sondern in einer hierzu parallelen Flüssigkeitstransportstrecke angeordnet, welche als Referenz dient. Außerdem hält er den Analyten vollständig zurück.

Ein weiterer Vorschlag, der eine wertvolle Ergänzung der vorgenannten Maßnahmen darstellt, bezieht sich auf die Startzone, deren Aufbau wesentlich ist für Handhabung und Funktion des Testträgers, wie im folgenden noch näher erläutert wird. Diesbezüglich schlägt die Erfindung vor, die Startzone aus einem nichtquellenden Faservlies herzustellen, das ein bei Raumtemperatur wasserunlösliches Bindemittel enthält.

Auch insoweit weicht die Erfindung von dem im vorbekannten Stand der Technik beschrittenen Weg grundsätzlich ab. In der deutschen Patentschrift 3 4 45 816 und in der europäischen Patentschrift 52 328 wird nämlich dargelegt, daß insbesondere Kunststoffschwämme oder Schichten aus hydrophilen Materialien, die aufgrund ihrer Quelleigenschaften eine besonders hohe Wasseraufnahme haben, geeignet sind. Dadurch werde sichergestellt, daß die Startzone beim Eintauchen in eine Probenflüssigkeit genügend Flüssigkeit aufnimmt, um die gesamte Flüssigkeitstransportstrecke des Testträgers zu füllen, auch wenn nach dem Eintauchen in die Probe keine weitere Flüssigkeit mehr zugeführt wird.

Überraschenderweise hat sich im Rahmen der vorliegenden Erfindung gezeigt, daß besonders gute Resultate mit einem verhältnismäßig hydrophoben Material erreicht werden, das zwar, bezogen auf sein Eigengewicht, eine geringere Flüssigkeitsaufnahme hat, das auf dem Testträger aber weitgehend vollständig leergesaugt wird.

Quantitativ läßt sich diese Eigenschaft als Flüssigkeitsretention ausdrücken. Diese wird im Rahmen der vorliegenden Erfindung dadurch bestimmt, daß eine Probe von 25 cm² Fläche auf ein Schwammtuch aufgelegt wird, welches erheblich größer als die Probe und bis zur Sättigung durchnäßt ist. Die dem Schwamm entzogene Wassermenge wird durch Wiegen bestimmt. Die nasse Probe wird auf einen Rundfilter Typ 2668/8 der Firma Schleicher & Schüll, Dassel, Bundesrepublik Deutschland von 158 mm Durchmesser gelegt und nach zwei Minuten entfernt. Die von dem Rundfilter aufgenommene Wassermenge wird wiederrum durch Wiegen bestimmte. Die Retention wird als prozentuales Verhältnis der in der Probe verbliebenen Wassermenge zu der ursprünglich aufgenommenen Wassermenge bestimmt. In diesem Sinne sind Materialien bevorzugt, die eine Flüssigkeitsretention von höchstens 25% für die Probenflüssigkeit haben.

Die Erfindung wird im folgenden anhand von in den Figuren schematisch dargestellten Ausführungsbeispielen näher erläutert; es zeigen:
- Fig. 1: eine Prinzipdarstellung der Zonenfolge eines erfindungsgemäßen Testträger
- Fig. 2: einen erfindungsgemäßen Testträger in perspektivischer Ansicht.

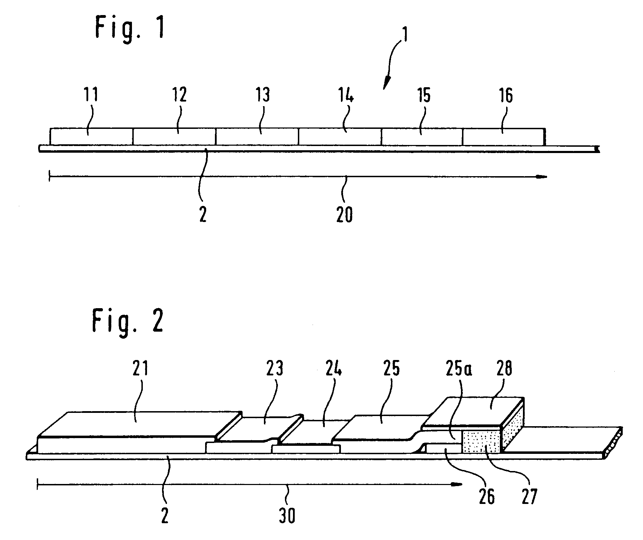

Figur 1 soll der Erläuterung verschiedener möglicher Testprinzipien und Anordnungen des Abfangreagenz auf dem Testträger dienen. Sie vernachläßigt deswegen konstruktive Einzelheiten und zeigt lediglich prinzipiell einen Testträger 1 mit einer Basisschicht 2, auf der nebeneinander, mit ihren Stirnflächen aneinanderstoßend,Testzonen 11-16 angeordnet sind. Die Testzonen bestehen bevorzugt jeweils aus verschiedenen saugfähigen Materialien (Papieren, Vliesen porösen Kunststoffschichten und dgl.),wobei der Fluidkontakt an den Stoßkanten durch ausreichend dichtes Aneinanderfügen der Schichten realisiert ist. Es kann jedoch auch zweckmäßig sein, daß die Schichten teilweise überlappen (vgl. Fig. 2) oder daß mehrere benachbarte Zonen aus dem gleichen Material einstückig hergestellt sind. Insgesamt bilden die Testzonen eine Flüssigkeitstransportstrecke 20, die von der Startzone 11 zu der Zielzone 16 führt.

Bei der Anwendung des Testträgers wird zunächst nur die Startzone 11 mit der Probenflüssigkeit kontaktiert, vorzugsweise, indem man den Testträger entsprechend eintaucht. Danach gibt die Startzone nach und nach ihren Flüssigkeitsgehalt an die Flüssigkeitstransportstrecke ab. Die Flüssigkeit chromatographiert aufgrund der in den Zonen 11 - 16 herrschenden Kapillarkräfte bis zu der Zielzone 16. Auf Einzelheiten der dabei wesentlichen Flüssigkeitstransporteigenschaften der Zonen wird im Zusammenhang mit Fig. 2 näher eingegangen.

An die Startzone 11 schließt sich im dargestellten Fall eine Probenaufgabezone 12 und eine Hilfsreagenzzone 13 an. Die Funktion der Probenaufgabezone wird weiter unten näher erläutert. Die Hilfsreagenzzone enthält einen Puffer, um den pH-Wert der Probe auf die Erfordernisse des Tests einzustellen. Gewünschtenfalls kann sie weitere Hilfsreagenzien, wie Netzmittel und dgl. enthalten. Allgemein können mehr oder weniger Zonen als dargestellt, je nach den Erfordernissen des Tests,vorhanden sein.

Je nach dem im Einzelfall zur Anwendung kommenden immunologischen Testprinzip, sind entsprechende Bestandteile des Reagenzsystems in den Zonen des Testträgers enthalten. Einige Beispiele werden im folgenden erläutert.

Bei einem kompetitiven Test zur Bestimmung eines in der Probe enthaltenen Antigens Ag kann beispielsweise die Zone 14 ein Konjugat des Antigens mit einem Markierungsenzym AgE in löslicher Form enthalten. Die Zone 15 enthält (als zweiten Bindungspartner) einen Antikörper in trägerfixierter Form Ak1(f).

Beim Durchströmen der Probe wird das AgE gelöst. Ag und AgE konkurrieren um Bindungsplätze an dem Ak1(f). Beim Weiterströmen der Flüssigkeit in die Zielzone 16 findet eine Trennung zwischen dem in der Zone 15 durch Bindung an das Ak1(f) fixierten AgE und dem freibleibenden AgE statt, weiches in die Zone 16 weiterströmt. Die in der Zone 15 gebundene AgE-Menge ist spezifisch und charakteristisch für die Analyse und kann dort nachgewiesen werden, indem man beispielsweise in einem weiteren Verfahrensschritt ein farbbildendes Substrat dem Enzyms zuführt und die Farbbildung in der Zone 15 beobachtet. Insoweit ist der Testverlauf konventionell (vgl. z.B. DE-PS 34 45 816).

Als Abfangreagenz befindet sich in der Zone 13 ein trägerfixierter dritter Bindungspartner Ak2(f). Er dient dazu, einen Teil des Probenantigens zu fixieren, bevor es in die Zone 14 mit dem Antigenkonjugat gelangt. Dadurch wird ein gewisser Teil des Antigens (allgemein gesprochen also des ersten Bindungspartners) der zu der markierten Spezies führenden Nachweisreaktion entzogen.

Wie erwähnt, müssen die Antikörper Ak1(f) und Ak2(f) jeweils mit dem gleichen Antigen spezifisch bindungsfähig sein. Vorzugsweise kann der gleiche Antikörper eingesetzt werden.

Im Falle eines nach dem immunenzymometrischen Testprinzip (IEMA) arbeitenden Testträgers zur Bestimmung eines in der Probe enthaltenen Antigens Ag enthält die Zone 14 ein lösliches Konjugat eines mit dem Antigen spezifisch bindenden Antikörpers mit einem Markierungsenzym AkE (zweiter Bindungspartner). Die Zone 15 enthält das Antigen in trägerfixierter Form (Ag(f), weiterer Bindungspartner). Beim Durchströmen der Probe wird das AkE gelöst und bildet mit dem Ag Komplexe Ag-AkE. Nicht komplexiertes AkE wird in der Zone 15 an das Ag(f) gebunden, während die Ag-AkE-Komplexe frei bleiben. Beim weiteren Transport der Flüssigkeit in die Zone 16 findet eine Trennung des gebundenen AkE und des für die Analyse charakteristischen komplexierten und deswegen frei beweglichen Ag-AkE statt. Letzteres kann in der Zone 16 nachgewiesen werden, wobei sich zweckmäßigerweise in der Zone 16 ein farbbildendes Substrat des Markierungsenzyms befindet. Auch dieser Testablauf ist bekannt.

Während bei dem kompetitiven Test der Nachweis wie oben beschrieben vorzugsweise in der Zone 15, also in der Zone mit dem fixierten zweiten Bindungspartner, stattfindet, dient beim immunenzymometrischen Test die der Fixierungszone 15 folgende Zone 16 als Nachweiszone.

Immunenzymometrische Tests zeichnen sich durch eine besonders hohe Empfindlichkeit und einfache Handhabung aus. Im Rahmen der vorliegenden Erfindung hat sich überraschenderweise gezeigt, daß Testträger, die nach diesem Prinzip arbeiten, auch für den direkten Nachweis verhältnismäßig hoch dosierter Analyten (Konzentration größer als 0,1 µmol/l) erfolgreich eingesetzt werden können, ohne daß dabei die Vorzüge bezüglich der leichten Handhabbarkeit verlorengehen.

Wiederum ist ein Abfangreagenz vorgesehen, welches ebenso wie bei dem zuvor beschriebenen kompetitiven Testprinzip in der Zone 13, also einer der Konjugatzone vorgelagerten Zone des Testträgers, löslich oder in trägerfixierter Form enthalten sein kann.
Besonders bevorzugt ist eine andere Variante, bei der sich das Abfangreagenz in löslicher Form mit dem Konjugat zusammen in der gleichen Zone (im Beispielsfall also in der Zone 14) befindet. In diesem Fall sollte der als Abfangreagenz verwendete Antikörper die gleiche Wirkspezifität wie der beim Konjugat verwendete Antikörper haben. Besonders geeignet ist deshalb ein identischer monoklonaler Antikörper, gegebenenfalls auch ein polyklonaler Antikörper mit einem sehr engen Epitopspektrum.

Bei dieser Variante der Erfindung konkurriert das Abfangreagenz, das keine Enzymmarkierung hat, mit dem Konjugat um die spezifischen Bindungsstellen an dem Analyten. Man erreicht dadurch eine im wesentlichen prozentuale "Verdünnung", d.h. der Anteil des Analyten, der durch das Abfangreagenz der Nachweisreaktion entzogen wird, entspricht näherungsweise dem Verhältnis zwischen Abfangreagenz und Konjugat.

Die Möglichkeit, das Abfangreagenz in freier Form vorzusehen ist nicht auf Fälle beschränkt, bei denen es in der gleichen Zone wie das Konjugat angeordnet ist. In ihrer vollen Allgemeinheit umfaßt die Erfindung vielmehr auch Testträger, bei denen das Abfangreagenz in löslicher Form in einer hierfür geeigneten, der Nachweiszone vorgelagerten, Zone der Flüssigkeitstransportstrecke enthalten ist.

Die Menge des Abfangreagenzes ist auf die spezifischen Erfordernisse abgestimmt. Theoretisch ist davon auszugehen, daß sie kleiner sein muß als die geringste nachzuweisende Menge des Analyten in der Probe (Nachweisgrenze). In der Praxis kommt es jedoch auf verschiedene Faktoren an. Hierzu gehört die Affinitätskonstante des verwendeten Antikörpers, die spezielle Konjugatstruktur und die Geschwindigkeit, mit der die Flüssigkeit durch die Zone, welche das Abfangreagenz enthält, strömt. Von Bedeutung ist auch, inwieweit die Bindung des Probenantigens an das Abfangreagenz vollständig ist. Aufgrund theoretischer Überlegungen wäre zu befürchten, daß hierin so starke Unsicherheiten liegen, daß eine erhebliche Verfälschung der Meßergebnisse zu befürchten ist. In der Praxis hat sich jedoch überraschenderweise gezeigt, daß man empirisch die Abfangreagenz-Menge so festlegen kann, daß eine immunologische Bestimmung hoch konzentrierter Parameter mit guter Genauigkeit möglich ist.

Vorzugsweise wird das saugfähige Schichtmaterial, welches die Testzone mit dem Abfangreagenz bildet, mit einer Tränklösung getränkt, in der das Abfangreagenz in einer molaren Konzentrattion enthalten ist, die etwa der gewünschten Nachweisgrenze der analytischen Bestimmung entspricht. Mindestens sollte die Konzentration das Abfangreagenz in der Tränklösung halb so groß sein, wie die gewünschte Nachweisgrenze. In absoluten Zahlen beträgt die Konzentration mindestens 10⁻⁸ mol/l, bevorzugt mindestens 10⁻⁷ mol/l.

Die Probe muß nicht notwendigerweise auf die Startzone aufgegeben werden. Es kann vielmehr auch eine spezielle Probenaufgabezone 12 vorgesehen sein. Dies ist vor allen Dingen dann zweckmäßig, wenn eine zusätzliche Probenverdünnung erreicht werden soll.

Dabei wird die Probe im Unterschuß auf die Probenaufgabezone 12 aufgegeben. "Im Unterschuß" ist in diesem Fall so zu verstehen, daß das aufgegebene Volumen geringer als die Saugkapazität der Probenaufgabezone ist. Anschließend wird die Startzone mit einem Eluationsmittel (zweckmäßigerweise Wasser oder Pufferlösung) kontaktiert.

Es wurde gefunden, daß dadurch eine Verdünnung erreicht werden kann. Der Grad der Verdünnung läßt sich durch das auf die Probenaufgabezone aufpipettierte Volumen und die Saugkapazität der Startzone beeinflussen. Ein besonderer Vorteil dieser Handhabungsweise besteht darin, daß man die Proben zu einem beliebigen Zeitpunkt aufdosieren und eintrocknen lassen kann. Die Eluation und Analyse erfolgt erst zu einem späteren Zeitpunkt. Dadurch können Serien von Teststreifentests angesammelt werden.

Wesentlich für die Verdünnungsfunktion des Probenaufgabefeldes ist, daß keine die Analyse störende Anreicherung der Probe in der Front der chromatographierenden Flüssigkeit entsteht. Deswegen wird zweckmäßigerweise für die Probenaufgabezone ein Material gewählt, daß aufgrund seiner besonderen Eigenschaften diese Bedingung erfüllt. Hierzu gehören folgende Wirkprinzipien:
a) Das Material ist so ausgewählt, daß der aufpipettierte Analyt an die Festphase schwach bindet und verzögert von dem Eluationsmittel eluiert wird.
b) Das Material der Probenauftragszone ist so strukturiert, daß durch Verwirbelung und/oder nichtlaminare Strömung eine Vermischung von Probe und Eluationsmittel erreicht wird.

Figur 2 zeigt einen Testträger, der in besonders einfacher Weise die Bestimmung verhältnismäßig hoch dosierter Parameter mit einem immunenzymometrischen Testprinzip erlaubt. Besonders geeignet ist er zur Bestimmung solcher Parameter in einer Probenflüssigkeit, die in verhältnismäßig großer Menge zur Verfügung steht, insbesondere im Urin.

Auf einer Basisschicht 2 sind im wesentlichen nebeneinander eine Startzone 21, eine Hilfsreagenzzone 23, eine Konjugatzone 24 und eine Fixierungszone 25 angeordnet. Eine Farbbildungszone 26 befindet sich zwischen dem Ende 25a der Fixierungszone 25 und der Basisschicht. Dabei wird das Ende 25a der Fixierungsschicht von einer aus steifem Kunststoffmaterial hergestellten Niederhalterschicht 28 gegen die Farbbildungsschicht 26 gedrückt. Die Niederhalterschicht ist mit einem Schmelzkleberstreifen 27 an der Basisschicht 2 befestigt.

In der Figur ist zu erkennen, daß die Schichten 21 - 25 jeweils die benachbarten Schichten geringfügig überlappen, um einen verbesserten Flüssigkeitskontakt an den Kanten der Zonen herzustellen. Im wesentlichen erfolgt jedoch der Flüssigkeitstransport in Längsrichtung des Testträgers und damit parallel zu der Oberfläche der Schichtmaterialien, aus der die Zonen hergestellt sind, entlang der Flüssigkeitstransportstrecke 30.

Bei der Benutzung wird der Testträger soweit in die Probenflüssigkeit eingetaucht, daß nur die Startzone 21 benetzt wird. Nur die Startzone 21 stellt also den Kontakt zur Probenflüssigkeit her. Sie muß daher so beschaffen sein, daß sie die Flüssigkeit spontan und vollständig aufnimmt und gut weiterleitet bzw. abgibt. Für einen Teststreifen der hier vorliegenden Art ist wichtig, daß alle der Startzone 21 nachgelagerten Zonen, die den eigentlichen Funktionsbereich des Testträgers bilden, ausreichend und reproduzierbar mit Flüssigkeit versorgt werden. Dies ließe sich zwar dadurch erreichen, daß man den Testträger in einem Gefäß mit der Probe stehenläßt, so daß ein ständiger Kontakt zu einem großen Flüssigkeitsvorrat vorhanden ist. Dies erschwert jedoch die Handhabung. Wünschenswert ist deshalb, daß die Startzone 21 innerhalb weniger Sekunden eine ausreichende Flüssigkeitsmenge aufnimmt und gemäß den Testanforderungen wieder abgibt, nachdem der Teststreifen aus der Probenflüssigkeit wieder herausgenommen wurde.

Im Gegensatz zu dem erwähnten älteren Vorschlag gemäß der EP-Patentanmeldung 52 328 besteht die Startzone im vorliegenden Fall erfindungsgemäß aus einem nichtquellenden Faserylies mit einem wasserunlöslichen Bindemittel.

Besonders bevorzugt als nichtquellendes Fasermaterial sind vollsynthetische Fasern, deren Anteil an dem Vlies bei mindestens 50%,liegen sollte. Bewährt haben sich insbesondere Polyamid und Polyester sowie Mischungen davon.

Als Bindemittel zur Verfestigung der Fasern kann vorteilhaft Polyvinylalkohol verwendet werden. Dies geschieht insbesondere in der Weise, daß bei der Herstellung des Vlieses in der Bütte die eigentlichen Vliesfasern mit Fasern aus Polyvinylalkohol gemischt werden. Sie werden dann, wie bei der Vliesherstellung üblich, auf eine Trocknungswalze aufgezogen. Bei einer Trocknungstemperatur von etwa 90°C - 150°C lösen sich die Polyvinylalkoholfasern und Bilden auf den übrigen Komponenten des Vlieses einen Überzug, der dem Vlies Festigkeit verleiht. Bevorzugt wird ein hochmolekularer, vollverseifter Polyvinylalkohol mit einem spezifischen Gewicht von 1,26 - 1,30 g/cm³ eingesetzt.

Besonders bevorzugte Polyesterfasern haben ein spezifisches Gewicht von ca. 1,17 g/cm³, eine Schnittlänge von 6 - 12 mm und eine Faserfeinheit von 1,7 - 3,3 dtex.

Soweit Polyamidfasern eingesetzt werden, haben sie bevorzugt ein spezifisches Gewicht von etwa 1,14 - 1,15 g/cm³, eine Schnittlänge von 4 - 6 mm und eine Faserfeinheit von 2,2 dtex.

Als zusätzlichen Bestandteil kann das Vlies Linters(aus der Grundwolle der Baumwollpflanze gewonnene Fasern, welche chemisch aufgeschlossen und gebleicht werden) enthalten.

Die weiteren Zonen enthalten die gleichen Reagenzien wie bei Figur 1 für den Fall eines immunenzymometrischen Tests beschrieben: Die Hilfsreagenzzone 23 enthält einen Puffer und gegebenenfalls weitere Hilfsreagenzien, die Konjugatzone 24 ein Enzymkonjugat eines mit dem Analyten spezifisch bindungsfähigen (zweiten) Bindungspartners, die Fixierungszone 25 festphasengebundenen Analyt (bzw. Analytanalogon) und die Farbbildungszone 26 ein farbbildendes Substrat des Markierungsenzyms.

Für den Fall der Bestimmung eines Antigens wird in der Konjugatzone 24 ein Konjugat eines entsprechenden Antikörpers und in der Fixierungszone 25 das gleiche Antigen oder ein anderes mit dem Antikörper der Schicht 24 bindungsfähiges Antigen verwendet. Soll ein Antikörper bestimmt werden, so ist, wie dem Fachmann bekannt ist, jeweils Antigen gegen Antikörper zu vertauschen.

Ebenfalls in Übereinstimmung mit Figur 1 ist ein Abfangreagenz vorgesehen, wobei dieses bevorzugt löslich und mit dem enzymatisch konjugierten Bindungspartner in der Schicht 24 identisch ist.

Der Testablauf entspricht der immunenzymometrischen Variante des Testträgers gemäß Figur 1 und muß daher hier nicht nochmals erläutert werden.

Für die Funktion des Testträgers nach Figur 2 sind auch die Flüssigkeitstransporteigenschaften der Schichtmaterialien, die die verschiedenen Zonen bilden, von besonderer Bedeutung.

Für einen optimalen Testablauf ist es erforderlich, daß die für die Analyse spezifische markierte Spezies in der Farbbildungszone 26 weitgehend homogen über deren gesamte Fläche vorliegt, so daß sich eine homogene Farbbildung ergibt. Im Fall eines immunenzymometrischen Tests soll also eine homogene Verteilung der für die Analyse charakteristischen Ag-AkE-Komplexe in der Farbbildungszone 26 erreicht werden.

Im Rahmen der vorliegenden Erfindung wurde gefunden, daß es in diesem Sinne vorteilhaft ist, wenn die Materialien, aus denen die Konjugatzone und die nächstfolgende Zone hergestellt sind, in ihren Saugeigenschaften so aufeinander abgestimmt sind, daß das Material der Konjugatzone die Flüssigkeit schneller transportiert als das Material der nächstfolgenden Zone.

Im dargestellten Beispielsfall bezieht sich dies auf die Konjugatzone 24 und die nächstfolgende Zone 25, die im allgemeinsten Fall nicht unbedingt eine Fixierungszone sein muß. Da beide Zonen im gleichen Flüssigkeitsstrom liegen, müssen sie im stationären Zustand, wenn beide Zonen gefüllt sind, die gleiche Flüssigkeitsmenge pro Zeiteinheit transportieren. Dennoch ergibt sich eine geringere Strömungsgeschwindigkeit, weil die Konjugatzone dünner ist und deswegen einen geringeren Strömungsquerschnitt hat als die nächstfolgende Zone.

Diese Maßnahme fördert die Homogenisierung des Konjugats. Abhängig von den Lösung- und Flüssigkeitstransportverhältnissen in der Konjugatzone 24 wird das Konjugat dort von der eintretenden Flüssigkeit im Sinne einer mehr oder weniger scharfen Konzentrationsfront gelöst. Dies entspricht dem normalen Verhalten einer saugfähigen Schicht bei der Flüssigkeitschromatographie. Zweck der hier diskutierten Maßnahme ist es, die daraus resultierenden Konzentrationsunterschiede auszugleichen. Die Konjugatzone wird zunächst verhältnismäßig schnell gefüllt, wobei sich das Konjugat noch wenig löst. Der weitere Flüssigkeitstransport erfolgt dann - entsprechend den Flüssigkeitstransporteigenschaften der nachfolgenden Zone - verhältnismäßig langsam. Während dieses langsamen Weiterfließens löst sich das Konjugat schon in der Zone 24 verhältnismäßig homogen. Außerdem bewirkt die langsame Strömung einen Equilibrierungseffekt innerhalg der Zone 25.

Im Falle des immunenzymometrischen Tests befindet sich, wie erläutert, eine Fixierungszone hinter der Konjugatzone, wobei sich im allgemeinsten Fall zwischen beiden Zonen weitere Zonen befinden können. Eine bevorzugte Maßnahme der Erfindung sieht nun vor, daß die Fixierungszone einen größeren Strömungsquerschnitt als die Konjugatzone hat.

Bei bekannten immunologischen Tests werden überwiegend feinporige Kunststoffmaterialien (Membranen) mit geringer Dicke als Fixierungsschicht verwendet, weil sie eine hohe Belegungsdichte der trägerfixierten immunologischen Reagenzien erlauben und die Flüssigkeit langsam durch sie hindurchströmt. Dadurch wird eine zuverlässige Fixierung des nicht komplexierten Konjugats aus der vorgeschalteten Konjugatschicht angestrebt. Dies ist von großer Bedeutung für die Meßgenauigkeit.

In Abweichung von diesem vorbekannten Weg wird im Rahmen der vorliegenden Erfindung nun vorzugsweise eine Fixierungsschicht verwendet, die auf Basis eines verhältnismäßig lockeren Trägermaterials, wie einem Papier, Gewebe oder (besonders bevorzugt) Vlies hergestellt ist. Das Schichtmaterial ist relativ dick, so daß die Fixierungszone einen größeren Strömungsquerschnitt als die Konjugatzone hat. Dadurch läßt sich auch hier eine sehr hohe Belegung mit Immunreagenzien erreichen, wobei die Herstellung erheblich weniger aufwendig als bei einer Membran ist. Durch die erwähnte Abstimmung von Konjugatzone und Fixierungszone ergibt sich gleichzeitig eine vergleichsweise langsame Strömung in der Fixierungsschicht, die für eine vollständige Bindung des überschüssigen Konjugats von Vorteil ist.

Die Homogenität der Farbbildung in der Schicht 26 wird bei dem dargestellten Testträger auch dadurch gefördert, daß die Farbbildungszone parallel zu einem Teil der Fixierungszone verläuft und in einem flächigen Flüssigkeitskontakt zu dieser steht. Zugleich ist die Farbbildungszone vorteilhaft so ausgebildet, daß die Flüssigkeit aus der Fixierungszone 25 nur verzögert in sie eindringt bzw. sich das Substrat in der Zone 26 derartig verzögert löst, daß sich die Fixierungszone 25 im wesentlichen vollständig gefüllt hat, bevor die Farbbildungsreaktion beginnt. Insoweit wird auf die deutschen Patentanmeldungen P 38 26 056.5 und P 38 26 057.3 der gleichen Anmelderin vom 30. Juli 1988 Bezug genommen.

Der weiteren Verdeutlichung der Erfindung dienen die folgenden Beispiele.

### Beispiel 1

Wasseraufnahme- und Abgabeeigenschaften von für die Startzone 11, 21 geeigneten Materialien.

Ein quellendes Schwammaterial gemäß der europäischen Patentanmeldung 52 328 wurde mit folgenden drei Materialien gemäß der vorliegenden Erfindung verglichen:
a) Mischvlies aus Polyamid und Polyester.
   Die Materialien werden in der Bütte zusammen mit Kuralon (Polyvinylalkohol hergestellt von Rohtex-Textil, Mönchengladbach, Bundesrepublik Deutschland) im Verhältnis Polyamid:Polyester:Kuralon= 30:20:2,6 gemischt und in bekannter Weise zu einem Vlies mit einem Flächengewicht von 160 g/m² und einer Dicke von 1,0 mm verarbeitet.
b) Reines Polyesterylies
   Polyester und Kuralon werden im Verhältnis 10:1 in der Bütte gemischt und zu einem Vlies mit einem Flächengewicht von 166 g/m² und einer Dicke von 1,0 mm verarbeitet.
c) Polyesterylies mit Linters
   Polyester, Linters und Kuralon werden im Gewichtsverhältnis 50:25:7,5 in der Bütte gemischt und zu einem Vlies mit einem Flächengewicht von 250 g/m² und einer Dicke von 1,4 mm verarbeitet.

Die folgende Tabelle zeigt das Retentionsverhalten solcher Vliese:

| | Wasseraufnahme | Wasserabgabe | Retention |
|---|---|---|---|
| Schwamm gemäß EP 0 052 328 | 5632 ml/m² | 3862 ml/m² | 31,4% |
| Vlies a) | 1825 ml/m² | 1636 ml/m² | 10,4% |
| Vlies b) | 1751 ml/m² | 1568 ml/m² | 10,5% |
| Vlies c) | 1866 ml/m² | 1548 ml/m² | 17,1% |

Man erkennt, daß Wasseraufnahme bei dem vorbekannten Schwamm am höchsten ist. Das gleiche gilt auch für die Wasserabgabe. Von besonderer Bedeutung für die Funktion des Testträgers ist jedoch die Retention, d.h. der Prozentsatz der in der jeweiligen Testzone unter bestimmten definierten Versuchsbedingungen zurückbleibenden Flüssigkeit. Aus dem Beispiel ersieht man, daß dieser Wert bei allen drei erfindungsgemäßen Zusammensetzungen erheblich besser ist, als bei dem vorbekannten Startzonenmaterial.

### Beispiel 2

Testträger für die Bestimmung von Albumin im Harn, insbesondere zum Nachweis einer Mikroalbuminurie.
Ein Testträger gemäß Figur 2 wird wie folgt hergestellt:

### Startzone 11:

Polyestervlies 1110 der Firma Binzer, Hatzfeld, Bundesrepublik Deutschland. Es handelt sich um ein reines Polyestervlies mit 10% Kuralon verfestigt. Die Dicke beträgt 1,0 - 1,2 mm, die Saugkapazität 1800 ml/m².

### Pufferzone 23:

Ein Vliesmaterial SL 4207 KA der Firma Kalff, Euskirchen, Bundesrepublik Deutschland, (bestehend aus 90% Polyester, 10% Zellwolle und geringen Mengen Acrylat) mit einer Dicke von 0,7 mm und einer Saugkapazität von 480 ml/m² wird mit folgender Lösung getränkt und anschließend getrocknet:
- 200mM Natriumphosphat pH 7,8
- 1% Rinder-Serumalbumin

### Konjugatzone 24:

Ein Glasfaservlies aus 100 Gewichtsteilen Glasfaser, verfestigt mit 10 Gewichtsteilen Kuralon in einer Dicke von 0,2 mm und mit einer Saugkapazität von 200 ml/m² wird mit folgender Lösung getränkt und anschließend getrocknet:
- 70mM Natriumphosphat pH 7,4
- 1% Trehalose
- 0,5% Rinder-Serumalbumin
- 6kU/l Konjugat aus β-Galactosidase und analytspezifischem Antikörper (IgG)
- 70mg/l analytspezifischer Antikörper (IgG) unmarkiert als Abfangreagenz

### Fixierungszone 25:

Ein Mischvlies aus Polyester, Baumwoll-Linters und Etadurin® im Gewichtsverhältnis 50:50:3 mit einer Dicke von 0,5 mm und einer Saugkapazität von 450 ml/m² wird mit folgender Lösung getränkt und anschließend 30 Minuten bei 50°C getrocknet
- 10 mM Natriumphosphat-Puffer, pH 7,5
- vernetztes Humanserum-Albumin in einer Konzentration von 200 mg/l Natriumphosphat-Puffer

### Das vernetzte Humanserum-Albumin wurde folgendermaßen hergestellt:

1,5 g Humanserum-Albumin wurden in 30 ml Kaliumphosphat-Puffer, 200 mM, pH 8,0 vorgelegt und innerhalb 2 Stunden mit 2,5 ml einer Lösung aus 50 mg Disuccinidyl-suberat/ml Dioxan versetzt. Nach Ablauf der Vernetzungsreaktion wurde gegen das 500fache Volumen Kaliumphosphat-Puffer, 20 mM, pH 7,2 dialysiert. Die hochmolekulare Fraktion mit einem Molekulargewicht von mehr als 650 000 Dalton wird an Superose 6® (Pharmacia, Freiburg, Bundesrepublik Deutschland) über Gelfiltration abgetrennt und nach Zugabe von 6 mg Saccharose/mg Protein lyophilisiert.

### Farbbildungszone 26:

Auf eine Polycarbonat-Folie von 0,1 mm Stärke wird ein löslicher Film mit folgender Rezeptur beschichtet:
- 4,5 g Mowiol 18/88 (Firma Hoechst, Frankfurt/M., Bundesrepublik Deutschland)
- 0,3 g ortho-Nitrophenol-β-galactosid gelöst in 50ml Wasser.

## Patentansprüche

1. Testträger zur analytischen Untersuchung einer Probenflüssigkeit mit Hilfe einer spezifischen Bindungsreaktion zweier bioaffiner Bindungspartner, von denen ein erster in der Probe und ein zweiter in dem Reagenzsystem des Testträgers enthalten ist,
mit mehreren auf einer Basisschicht (2) im wesentlichen nebeneinander angeordneten kapillaraktiven Testzonen (11-16, 21-25), die in Fluidkontakt zueinander stehen, so daß sie eine Flüssigkeitstransportstrecke (20,30) bilden, entlang der eine Flüssigkeit, durch Kapillarkräfte getrieben, von einer Startzone (11,21) ausgehend, strömt,
wobei zwischen dem ersten Bindungspartner und dem den zweiten Bindungspartner enthaltenden Reagenzsystem eine Reaktion abläuft, die zu einer für die gewünschte Analyse spezifischen markierten Spezies führt und die spezifische markierte Spezies in einer Nachweiszone (16,26) nachgewiesen wird,
**dadurch gekennzeichnet,** daß
in der zum analytischen Nachweis insbesondere eines in verhältnismäßig hoher Konzentration vorliegenden ersten Bindungspartners dienenden Flüssigkeitstransportstrecke ein als Abfangreagenz wirkender für den ersten Bindungspartner spezifischer dritter Bindungspartner vorgesehen ist, der in einer Zone der Flüssigkeitstransportstrecke (20,30) so angeordnet und bezüglich der eingesetzten Menge so bemessen ist, daß er einen Teil des ersten Bindungspartners bindet und dadurch der zu der spezifischen markierten Spezies führenden Reaktion entzieht.

2. Testträger nach Anspruch 1, **dadurch gekennzeichnet,** daß das Abfangrcagenz löslich ist.

3. Testträger nach Anspruch 1, **dadurch gekennzeichnet,** daß er eine Konjugatzone (14) einschließt, die ein lösliches Konjugat des zweiten Bindungspartners mit einer Markierung enthält und das Abfangreagenz im vorderen Teil der Flüssigkeitstransportstrecke bis einschließlich der Konjugatzone angeordnet ist.

4. Testträger nach Anspruch 3, **dadurch gekennzeichnet,** daß das Abfangreagenz in der Konjugatzone angeordnet ist.

5. Testträger nach Anspruch 3 oder 4, **dadurch gekennzeichnet,** daß die Materialien, aus denen die Konjugatzone (14) und die nächstfolgende Zone hergestellt sind, in ihren Saugeigenschaften so aufeinander abgestimmt sind, daß das Material der Konjugatzone die Flüssigkeit schneller transportiert als das Material der nächstfolgenden Zone.

6. Testträger nach Anspruch 3, **dadurch gekennzeichnet,** daß in der Flüssigkeitstransportstrecke (20,30) hinter der Konjugatzone (14,24) eine Fixierungszone (15,25) angeordnet ist, die einen zu dem ersten Bindungspartner analogen Bindungspartner des zweiten Bindungspartners in trägerfixierter Form enthält.

7. Testträger nach Anspruch 6, **dadurch gekennzeichnet,** daß die Fixierungszone (25) einen größeren Strömungsquerschnitt als die Konjugatzone (24) hat.

8. Testträger nach Anspruch 1, **dadurch gekennzeichnet,** daß das Abfangreagenz in einer auf der Flüssigkeitstransportstrecke vor der den zweiten Bindungspartner enthaltenden Zone (14 bzw. 15) angeordneten Testzone (13) trägerfixiert ist.

9. Testträger nach einem der Ansprüche 1 - 8, **dadurch gekennzeichnet,** daß der zweite Bindungspartner und dritte Bindungspartner gleich sind.

10. Testträger nach Anspruch 1, **dadurch gekennzeichnet,** daß
die Startzone (11,12) aus einem nicht quellenden Faservlies mit einem wasserunlöslichen Bindemittel besteht.

11. Testträger nach Anspruch 10, **dadurch gekennzeichnet,** daß das Faservlies eine Retention von höchstens 25% für die Probenflüssigkeit hat.

12. Testträger nach Anspruch 11, **dadurch gekennzeichnet,** daß das Faservlies mindestens 50% vollsynthetische Fasern, vorzugsweise Polyamid oder Polyester enthält.

13. Testträger nach Anspruch 12, **dadurch gekennzeichnet,** daß das Faservlies Polyvinylalkohol enthält.

14. Verfahren zur analytischen Untersuchung einer Probenflüssigkeit mit einem Testträger nach Anspruch 1, **dadurch gekennzeichnet,** daß die spezifische Bindungsreaktion des in der Probe enthaltenen ersten Bindungspartners mit dem als Abfangreagenz wirkenden dritten Bindungspartner spätestens gleichzeitig mit der spezifischen Bindungsreaktion des ersten Bindungspartners mit dem zweiten Bindungspartner stattfindet.

15. Verfahren nach Anspruch 14, **dadurch gekennzeichnet,** daß der Testträger in der Flüssigkeitstransportstrecke hinter der Startzone eine Probenaufgabezone aufweist und die Probe auf die Probenaufgabezone in einer Menge aufgegeben wird, die geringer ist als die Saugkapazität der Probenaufgabezone.

## Claims

1. Test carrier for analytical investigation of a sample liquid with the help of a specific binding reaction of two bioaffine binding partners, the first of which is contained in the sample and the second in a reagent system of the test carrier, with several capillary-active test zones (11-16, 21-25) arranged substantially next to one another on a base layer (2) which are in liquid contact with one another so that they form a liquid transport path (20,30) along which a liquid flows, driven by capillary forces, from a start zone (11,21), whereby a reaction takes place between the first binding partner and the reagent system containing the second binding partner, which leads to a labelled species specific for the desired analysis and the specific labelled species is detected in a detection zone (16,26), characterised in that, in the liquid transport path serving for the analytical detection especially of a first binding partner present in comparatively high concentration, there is provided a third binding partner, acting as capturing reagent, specific for the first binding partner which is so arranged in a zone of the liquid transport path (20,30) and is so measured with regard to the amount used that it binds a part of the first binding partner and thereby removes it from the reaction leading to the specific labelled species.

2. Test carrier according to claim 1, characterised in that the capturing reagent is soluble.

3. Test carrier according to claim 1, characterised in that it includes a conjugate zone (14) which contains a soluble conjugate of the second binding partner with a label and the capturing reagent is arranged in the front part of the liquid transport path up to and including the conjugate zone.

4. Test carrier according to claim 3, characterised in that the capturing zone is arranged in the conjugate zone.

5. Test carrier according to claim 3 or 4, characterised in that the materials from which the conjugate zone (14) and the following zone are produced are so adapted to one another in their absorbent properties that the material of the conjugate zone transports the liquid more quickly than the material of the following zone.

6. Test carrier according to claim 3, characterised in that, in the liquid transport path (20,30), after the conjugate zone (14,24), there is provided a fixing zone (15,25) which contains, in carrier-fixed form, a binding partner of the second binding partner analogous to the first binding partner.

7. Test carrier according to claim 6, characterised in that the fixing zone (25) has a larger flow cross-section than the conjugate zone (24).

8. Test carrier according to claim 1, characterised in that the capturing reagent is carrier-fixed in a test zone (13) arranged on the liquid transport path in front of the zone (14 or 15) containing the second binding partner.

9. Test carrier according to one of claims 1 - 8, characterised in that the second binding partner and the third binding partner are the same.

10. Test carrier according to claim 1, characterised in that the start zone (11,12) consists of a non-swelling fibre fleece with a water-insoluble binding agent.

11. Test carrier according to claim 10, characterised in that the fibre fleece has a retention for the sample liquid of at most 25%.

12. Test carrier according to claim 11, characterised in that the fibre fleece contains at least 50% of completely synthetic fibres, preferably polyamide or polyester.

13. Test carrier according to claim 12, characterised in that the fibre fleece contains polyvinyl alcohol.

14. Process for the analytical investigation of a sample liquid with a test carrier according to claim 1, characterised in that the specific binding reaction of the first binding partner contained in the sample with the third binding partner acting as capturing reagent takes place simultaneously with the specific binding reaction of the first binding partner with the second binding partner.

## Revendications

1. Support de test pour la détermination analytique d'un échantillon liquide, à l'aide d'une réaction de liaison spécifique entre deux partenaires de liaison bioaffine, dont le premier se trouve dans l'échantillon et le second, dans le système de réactifs du support de test, comportant, sur une couche de base (2), plusieurs zones de test (11-16, 21-25) essentiellement juxtaposées, ayant une activité capillaire, qui sont en contact fluidique entre elles, de façon à former une voie de transport de liquide (20, 30) suivant laquelle s'écoule un liquide par capillarité, à partir d'une zone de départ (11, 21), tandis qu'entre le premier partenaire de liaison et le système de réactifs contenant le second partenaire de liaison, se déroule une réaction qui conduit à une espèce marquée spécifique pour l'analyse voulue, et l'espèce marquée spécifique est détectée dans une zone de détection (16, 26), caractérisé en ce que dans la voie de transport de liquide servant en particulier à la détection analytique d'un premier partenaire de liaison présent en une concentration relativement élevée, il est prévu un troisième partenaire de liaison, agissant comme réactif initial, de façon spécifique pour le premier partenaire réactionnel, qui est disposé dans une zone de la voie de transport de liquide (20, 30) et dosée par rapport à la quantité mise en oeuvre de manière à fixer une partie du premier partenaire de liaison et ainsi, déclenche la réaction conduisant à la formation de l'espèce marquée spécifique.

2. Support de test selon la revendication 1, caractérisé en ce que le réactif initial est soluble.

3. Support de test selon la revendication 1, caractérisé en ce qu'il comprend une zone conjuguée (14) qui contient un conjugué soluble du deuxième partenaire de liaison avec un marqueur, et que le réactif initial est disposé dans la première partie de la voie de transport de liquide, jusqu'à et comprise la zone du conjugué.

4. Support de test selon la revendication 3, caractérisé en ce que le réactif initial est disposé dans la zone du conjugué.

5. Support de test selon la revendication 3 ou 4, caractérisé en ce que les substances constituant la zone du conjugué (14) et la zone suivante sont ajustées entre elles, du point de vue de leurs propriétés d'absorption, de façon que la substance de la zone du conjugué transporte le liquide plus rapidement que la substance de la zone suivante.

6. Support de test selon la revendication 3, caractérisé en ce que dans la voie de transport de liquide (20, 30), après la zone du conjugué (14, 24), se trouve une zone de fixation (15, 25) qui contient un partenaire de liaison du deuxième partenaire de liaison, analogue au premier partenaire de liaison, sous forme fixée.

7. Support de test selon la revendication 6, caractérisé en ce que la zone de fixation (25) présente une section transversale d'écoulement qui est supérieure à celle de la zone du conjugué (24).

8. Support de test selon la revendication 1, caractérisé en ce que le réactif initial est fixé sur le support dans une zone de test (13) disposée, dans la voie de transport de liquide, avant la zone (14 et/ou 15) contenant le deuxième partenaire de liaison.

9. Support de test selon l'une quelconque des revendications 1-8, caractérisé en ce que le deuxième partenaire de liaison et le troisième partenaire de liaison sont identiques.

10. Support de test selon la revendication 1, caractérisé en ce que la zone initiale (11, 12) est constituée d'une nappe de fibres comportant une liant insoluble dans l'eau.

11. Support de test selon la revendication 10, caractérisé en ce que la nappe de fibres présente une rétention de 25 % au plus pour l'échantillon liquide.

12. Support de test selon la revendication 11, caractérisé en ce que la nappe de fibres comporte au moins 50 % de fibres entièrement synthétiques, de préférence en polyamide ou polyester.

13. Support de test selon la revendication 12, caractérisé en ce que la nappe de fibres contient du poly(alcool vinylique).

14. Procédé de détermination analytique d'un échantillon liquide à l'aide d'un support de test selon la revendication 1, caractérisé en ce que la réaction de liaison spécifique du premier partenaire de liaison, contenu dans l'échantillon, avec le troisième partenaire de liaison, agissant comme réactif initial, se déroule au plus tard en même temps que la réaction de liaison spécifique du premier partenaire de liaison avec le deuxième partenaire de liaison.
